# EUROPEAN PATENT APPLICATION

(11) **EP 4 663 654 A1**
(43) Date of publication of application: **17.12.2025**
(21) Application number: 24752634.6
(22) Date of filing: 05.01.2024
(51) Int. Cl.: C07K 14/605, A61K 38/00, A61P 3/10, A61P 3/04, A61P 3/06

(54) **POLYPEPTIDE AND DERIVATIVE THEREOF, COMPOSITION, AND USE THEREOF**

(30) Priority: 10.02.2023 CN 202310158334
(71) Applicant: Hangzhou Sciwind Biosciences Co., Ltd., Hangzhou, Zhejiang 310015 (CN); Sciwind Biosciences (Beijing) Co., Ltd., Beijing 100176 (CN)
(72) Inventor: PAN, Hai, Beijing 100176 (CN); LI, Yan, Beijing 100176 (CN); ZOU, Haixia, Beijing 100176 (CN); HAO, Sujuan, Beijing 100176 (CN)
(74) Representative: Snaith, James Michael
(86) International application number: PCT/CN2024/070964
(87) International publication number: WO 2024/164776

(57) **Abstract**

Provided in the present application is a polypeptide and a derivative of the polypeptide or a pharmaceutically acceptable salt thereof. The present application also provides a pharmaceutical composition comprising the polypeptide or the derivative or a pharmaceutically acceptable salt thereof described in the present application, and a pharmaceutically acceptable excipient. Use of the polypeptide, the derivative of the polypeptide or a pharmaceutically acceptable salt thereof, or the composition as described above in the field of disease prevention/treatment is also provided.

## Description

### TECHNICAL FIELD

The present application relates to the technical field of polypeptides, specifically, a polypeptide and its derivatives, a composition comprising the same, and use thereof.

### BACKGROUND

Amylin, also known as an islet amyloid polypeptide (IAPP, amylin, or hAMY1-37), is a polypeptide hormone consisting of 37 amino acids serving as a member of the calcitonin protein family that also includes calcitonin (CT), calcitonin gene related peptides (CGRP), and human adrenomedullin (hAM) and precursors thereof. Amylin is co-secreted with insulin in islet β cells and is lacking in patients with diabetes. It functions in several different organ systems primarily by virtue of amylin receptors 1-3 (AMYRs 1-3). Amylin can inhibit secretion of glucagon, delay gastric emptying, give a signal of satiety, and suppress appetite. Clinical studies have shown that amylin receptor agonists may be used for treating overweight, obesity, type 1 diabetes and/or type 2 diabetes. However, amylin has some shortcomings such as a strong tendency towards fibrillation, a short half-life *in vivo,* and a chemical instability at pH 7. Therefore, natural amylin is not suitable for use as an active pharmaceutical ingredient.

A number of amylin analogs or derivatives are known in the prior art to attempt to make up for some known deficiencies of human amylin. A successful example is an amylin analog named Pramlintide (Trade name Symlin) that has been approved by FDA for use in type I and type II diabetes. However, Pramlintide has a half-life of less than an hour and is for mealtime administration, and thus a patient needs to take this medicine for treatment multiple times a day. In addition, Pramlintide is formulated at pH 4 because it fibrillates at pH 7 and becomes ineffective due to precipitation. Therefore, there is still a need for a novel amylin analog or derivative with increased chemical stability, increased metabolic stability and/or reduced tendency for fibrillation. Particularly, an amylin analog or derivative that is stable in a wider range of pH is needed. In addition, there is also a need for a novel amylin analog or derivative whose efficacy is enhanced by increasing its potency, improving its therapeutic effect and/or prolonging its half-life so as to lower dosing frequency and improve patient compliance. Pramlintide currently available in the market has a half-life of less than an hour and needs to be administered multiple times a day for treatment, and cannot be prepared into a single liquid formulation together with other drugs under neutral conditions.

### SUMMARY OF THE INVENTION

The technical problem to be addressed by the present application is to figure out a solution to the deficiencies as mentioned in the BACKGROUND by providing a polypeptide and its derivatives, a composition comprising the same, and uses thereof.

Provided in the present application is a derivative of a polypeptide or a pharmaceutically acceptable salt thereof comprising an amino acid sequence of ASX₃LS TAX₈X₉X₁₀ RLADF LRHX₁₉X₂₀ X₂₁X₂₂X₂₃X₂₄X₂₅ ILPPT NVGSN TX₃₇-NH₂;
wherein: X₃ is E or Q; X₈ is A or V; X₉ is L or T; X₁₀ is G or Q; X₁₉ is S or F; X₂₀ is S or T; X₂₁ is N, Q, E, S, T, A, G, H, K, R or D; X₂₂ is N, Q, E, S, NMeAsn, αMeAsn, NMeAsp or R; X₂₃ is L or D; X₂₄ is K or R; X₂₅ is P or D; and X₃₇ is P or trans-Hyp; and the derivative comprises a fatty acid-containing side chain linked to the N terminus of the polypeptide.

In some embodiments of the present application, X₃ is E or Q; X₈ is A or V; X₉ is L or T; X₁₀ is G or Q; X₁₉ is S or F; X₂₀ is S or T; X₂₁ is N or D; X₂₂ is N or R; X₂₃ is L or D; X₂₄ is K or R; X₂₅ is P or D; and X₃₇ is P or trans-Hyp.

In some embodiments of the present application, X₃ is E, X₈ is A or V; X₉ is L or T; X₁₀ is G or Q; X₁₉ is S or F; X₂₀ is S or T; X₂₁ is N or D; X₂₂ is N or R; X₂₃ is L or D; X₂₄ is K or R; X₂₅ is P or D; and X₃₇ is P or trans-Hyp.

In some embodiments of the present application, X₃ is Q, X₈ is A or V; X₉ is L or T; X₁₀ is G or Q; X₁₉ is S or F; X₂₀ is S or T; X₂₁ is N or D; X₂₂ is N or R; X₂₃ is L or D; X₂₄ is K or R; X₂₅ is P or D; and X₃₇ is P or trans-Hyp.

In some embodiments of the present application, X₈ is A, X₃ is E or Q; X₉ is L or T; X₁₀ is G or Q; X₁₉ is S or F; X₂₀ is S or T; X₂₁ is N or D; X₂₂ is N or R; X₂₃ is L or D; X₂₄ is K or R; X₂₅ is P or D; and X₃₇ is P or trans-Hyp.

In some embodiments of the present application, X₈ is V, X₃ is E or Q; X₉ is L or T; X₁₀ is G or Q; X₁₉ is S or F; X₂₀ is S or T; X₂₁ is N or D; X₂₂ is N or R; X₂₃ is L or D; X₂₄ is K or R; X₂₅ is P or D; and X₃₇ is P or trans-Hyp.

In some embodiments of the present application, X₉ is L, X₃ is E or Q; X₈ is A or V; X₁₀ is G or Q; X₁₉ is S or F; X₂₀ is S or T; X₂₁ is N or D; X₂₂ is N or R; X₂₃ is L or D; X₂₄ is K or R; X₂₅ is P or D; and X₃₇ is P or trans-Hyp.

In some embodiments of the present application, X₉ is T, X₃ is E or Q; X₈ is A or V; X₁₀ is G or Q; X₁₉ is S or F; X₂₀ is S or T; X₂₁ is N or D; X₂₂ is N or R; X₂₃ is L or D; X₂₄ is K or R; X₂₅ is P or D; and X₃₇ is P or trans-Hyp.

In some embodiments of the present application, X₁₀ is G, X₃ is E or Q; X₈ is A or V; X₉ is L or T; X₁₉ is S or F; X₂₀ is S or T; X₂₁ is N or D; X₂₂ is Nor R; X₂₃ is L or D; X₂₄ is K or R; X₂₅ is P or D; and X₃₇ is P or trans-Hyp.

In some embodiments of the present application, X₁₀ is Q; X₃ is E or Q; X₈ is A or V; X₉ is L or T; X₁₉ is S or F; X₂₀ is S or T; X₂₁ is N or D; X₂₂ is Nor R; X₂₃ is L or D; X₂₄ is K or R; X₂₅ is P or D; and X₃₇ is P or trans-Hyp.

In some embodiments of the present application, X₁₉ is S, X₃ is E or Q; X₈ is A or V; X₉ is L or T; X₁₀ is G or Q; X₂₀ is S or T; X₂₁ is N or D; X₂₂ is N or R; X₂₃ is L or D; X₂₄ is K or R; X₂₅ is P or D; and X₃₇ is P or trans-Hyp.

In some embodiments of the present application, X₁₉ is F; X₃ is E or Q; X₈ is A or V; X₉ is L or T; X₁₀ is G or Q; X₂₀ is S or T; X₂₁ is N or D; X₂₂ is N or R; X₂₃ is L or D; X₂₄ is K or R; X₂₅ is P or D; and X₃₇ is P or trans-Hyp.

In some embodiments of the present application, X₂₀ is S; X₃ is E or Q; X₈ is A or V; X₉ is L or T; Xiₒ is G or Q; X₁₉ is S or F; X₂₁ is N or D; X₂₂ is N or R; X₂₃ is L or D; X₂₄ is K or R; X₂₅ is P or D; and X₃₇ is P or trans-Hyp.

In some embodiments of the present application, X₂₀ is T; X₃ is E or Q; X₈ is A or V; X₉ is L or T; Xiₒ is G or Q; X₁₉ is S or F; X₂₁ is N or D; X₂₂ is N or R; X₂₃ is L or D; X₂₄ is K or R; X₂₅ is P or D; and X₃₇ is P or trans-Hyp.

In some embodiments of the present application, X₂₁ is N, X₃ is E or Q; X₈ is A or V; X₉ is L or T; X₁₀ is G or Q; X₁₉ is S or F; X₂₀ is S or T; X₂₂ is N or R; X₂₃ is L or D; X₂₄ is K or R; X₂₅ is P or D; and X₃₇ is P or trans-Hyp.

In some embodiments of the present application, X₂₁ is D; X₃ is E or Q; X₈ is A or V; X₉ is L or T; X₁₀ is G or Q; X₁₉ is S or F; X₂₀ is S or T; X₂₂ is N or R; X₂₃ is L or D; X₂₄ is K or R; X₂₅ is P or D; and X₃₇ is P or trans-Hyp.

In some embodiments of the present application, X₂₁ is Q; X₃ is E or Q; X₈ is A or V; X₉ is L or T; X₁₀ is G or Q; X₁₉ is S or F; X₂₀ is S or T; X₂₂ is N or R; X₂₃ is L or D; X₂₄ is K or R; X₂₅ is P or D; and X₃₇ is P or trans-Hyp.

In some embodiments of the present application, X₂₁ is E; X₃ is E or Q; X₈ is A or V; X₉ is L or T; X₁₀ is G or Q; X₁₉ is S or F; X₂₀ is S or T; X₂₂ is N or R; X₂₃ is L or D; X₂₄ is K or R; X₂₅ is P or D; and X₃₇ is P or trans-Hyp.

In some embodiments of the present application, X₂₁ is S; X₃ is E or Q; X₈ is A or V; X₉ is L or T; X₁₀ is G or Q; X₁₉ is S or F; X₂₀ is S or T; X₂₂ is N or R; X₂₃ is L or D; X₂₄ is K or R; X₂₅ is P or D; and X₃₇ is P or trans-Hyp.

In some embodiments of the present application, X₂₁ is T; X₃ is E or Q; X₈ is A or V; X₉ is L or T; X₁₀ is G or Q; X₁₉ is S or F; X₂₀ is S or T; X₂₂ is N or R; X₂₃ is L or D; X₂₄ is K or R; X₂₅ is P or D; and X₃₇ is P or trans-Hyp.

In some embodiments of the present application, X₂₁ is A; X₃ is E or Q; X₈ is A or V; X₉ is L or T; X₁₀ is G or Q; X₁₉ is S or F; X₂₀ is S or T; X₂₂ is N or R; X₂₃ is L or D; X₂₄ is K or R; X₂₅ is P or D; and X₃₇ is P or trans-Hyp.

In some embodiments of the present application, X₂₁ is G; X₃ is E or Q; X₈ is A or V; X₉ is L or T; X₁₀ is G or Q; X₁₉ is S or F; X₂₀ is S or T; X₂₂ is N or R; X₂₃ is L or D; X₂₄ is K or R; X₂₅ is P or D; and X₃₇ is P or trans-Hyp.

In some embodiments of the present application, X₂₁ is H; X₃ is E or Q; X₈ is A or V; X₉ is L or T; X₁₀ is G or Q; X₁₉ is S or F; X₂₀ is S or T; X₂₂ is N or R; X₂₃ is L or D; X₂₄ is K or R; X₂₅ is P or D; and X₃₇ is P or trans-Hyp.

In some embodiments of the present application, X₂₁ is K; X₃ is E or Q; X₈ is A or V; X₉ is L or T; X₁₀ is G or Q; X₁₉ is S or F; X₂₀ is S or T; X₂₂ is N or R; X₂₃ is L or D; X₂₄ is K or R; X₂₅ is P or D; and X₃₇ is P or trans-Hyp.

In some embodiments of the present application, X₂₁ is R; X₃ is E or Q; X₈ is A or V; X₉ is L or T; X₁₀ is G or Q; X₁₉ is S or F; X₂₀ is S or T; X₂₂ is N or R; X₂₃ is L or D; X₂₄ is K or R; X₂₅ is P or D; and X₃₇ is P or trans-Hyp.

In some embodiments of the present application, X₂₂ is N, X₃ is E or Q; X₈ is A or V; X₉ is L or T; X₁₀ is G or Q; X₁₉ is S or F; X₂₀ is S or T; X₂₁ is N or D; X₂₃ is L or D; X₂₄ is K or R; X₂₅ is P or D; and X₃₇ is P or trans-Hyp.

In some embodiments of the present application, X₂₂ is R, X₃ is E or Q; X₈ is A or V; X₉ is L or T; X₁₀ is G or Q; X₁₉ is S or F; X₂₀ is S or T; X₂₁ is N or D; X₂₃ is L or D; X₂₄ is K or R; X₂₅ is P or D; and X₃₇ is P or trans-Hyp.

In some embodiments of the present application, X₂₂ is Q, X₃ is E or Q; X₈ is A or V; X₉ is L or T; X₁₀ is G or Q; X₁₉ is S or F; X₂₀ is S or T; X₂₁ is N or D; X₂₃ is L or D; X₂₄ is K or R; X₂₅ is P or D; and X₃₇ is P or trans-Hyp.

In some embodiments of the present application, X₂₂ is E, X₃ is E or Q; X₈ is A or V; X₉ is L or T; X₁₀ is G or Q; X₁₉ is S or F; X₂₀ is S or T; X₂₁ is N or D; X₂₃ is L or D; X₂₄ is K or R; X₂₅ is P or D; and X₃₇ is P or trans-Hyp.

In some embodiments of the present application, X₂₂ is S, X₃ is E or Q; X₈ is A or V; X₉ is L or T; X₁₀ is G or Q; X₁₉ is S or F; X₂₀ is S or T; X₂₁ is N or D; X₂₃ is L or D; X₂₄ is K or R; X₂₅ is P or D; and X₃₇ is P or trans-Hyp.

In some embodiments of the present application, X₂₂ is NMeAsn, X₃ is E or Q; X₈ is A or V; X₉ is L or T; X₁₀ is G or Q; X₁₉ is S or F; X₂₀ is S or T; X₂₁ is N or D; X₂₃ is L or D; X₂₄ is K or R; X₂₅ is P or D; and X₃₇ is P or trans-Hyp.

In some embodiments of the present application, X₂₂ is αMeAsn, X₃ is E or Q; X₈ is A or V; X₉ is L or T; X₁₀ is G or Q; X₁₉ is S or F; X₂₀ is S or T; X₂₁ is N or D; X₂₃ is L or D; X₂₄ is K or R; X₂₅ is P or D; and X₃₇ is P or trans-Hyp.

In some embodiments of the present application, X₂₂ is NMeAsp, X₃ is E or Q; X₈ is A or V; X₉ is L or T; X₁₀ is G or Q; X₁₉ is S or F; X₂₀ is S or T; X₂₁ is N or D; X₂₃ is L or D; X₂₄ is K or R; X₂₅ is P or D; and X₃₇ is P or trans-Hyp.

In some embodiments of the present application, X₂₃ is L, X₃ is E or Q; X₈ is A or V; X₉ is L or T; X₁₀ is G or Q; X₁₉ is S or F; X₂₀ is S or T; X₂₁ is N or D; X₂₂ is N or R; X₂₄ is K or R; X₂₅ is P or D; and X₃₇ is P or trans-Hyp.

In some embodiments of the present application, X₂₃ is D; X₃ is E or Q; X₈ is A or V; X₉ is L or T; X₁₀ is G or Q; X₁₉ is S or F; X₂₀ is S or T; X₂₁ is N or D; X₂₂ is N or R; X₂₄ is K or R; X₂₅ is P or D; and X₃₇ is P or trans-Hyp.

In some embodiments of the present application, X₂₄ is K, X₃ is E or Q; X₈ is A or V; X₉ is L or T; X₁₀ is G or Q; X₁₉ is S or F; X₂₀ is S or T; X₂₁ is N or D; X₂₂ is N or R; X₂₃ is L or D; X₂₅ is P or D; and X₃₇ is P or trans-Hyp.

In some embodiments of the present application, X₂₄ is R; X₃ is E or Q; X₈ is A or V; X₉ is L or T; X₁₀ is G or Q; X₁₉ is S or F; X₂₀ is S or T; X₂₁ is N or D; X₂₂ is N or R; X₂₃ is L or D; X₂₅ is P or D; and X₃₇ is P or trans-Hyp.

In some embodiments of the present application, X₂₅ is P, X₃ is E or Q; X₈ is A or V; X₉ is L or T; X₁₀ is G or Q; X₁₉ is S or F; X₂₀ is S or T; X₂₁ is N or D; X₂₂ is N or R; X₂₃ is L or D; X₂₄ is K or R; and X₃₇ is P or trans-Hyp.

In some embodiments of the present application, X₂₅ is D, X₃ is E or Q; X₈ is A or V; X₉ is L or T; X₁₀ is G or Q; X₁₉ is S or F; X₂₀ is S or T; X₂₁ is N or D; X₂₂ is N or R; X₂₃ is L or D; X₂₄ is K or R; and X₃₇ is P or trans-Hyp.

In some embodiments of the present application, X₃₇ is P, X₃ is E or Q; X₈ is A or V; X₉ is L or T; X₁₀ is G or Q; X₁₉ is S or F; X₂₀ is S or T; X₂₁ is N or D; X₂₂ is N or R; X₂₃ is L or D; X₂₄ is K or R; and X₂₅ is P or D.

In some embodiments of the present application, X₃₇ is trans-Hyp, X₃ is E or Q; X₈ is A or V; X₉ is L or T; Xiₒ is G or Q; X₁₉ is S or F; X₂₀ is S or T; X₂₁ is N or D; X₂₂ is N or R; X₂₃ is L or D; X₂₄ is K or R; and X₂₅ is P or D.

In some embodiments of the present application, X₃ is Q; preferably, X₈X₉X₁₀ is selected from any of ALG, VLG, VTQ, ATQ, and VLQ; and further preferably, X₈X₉X₁₀ is ATQ; and more preferably, X₁₉ is F, X₂₀ is T, X₂₃ is D, X₂₄ is R, X₂₅ is D, and X₃₇ is P; and further preferably, X₂₁ is D and/or X₂₂ is R.

In some embodiments of the present application, X₃ is Q; preferably, X₈X₉X₁₀ is selected from any of ALG, VLG, VTQ, ATQ, and VLQ; and further preferably, X₈X₉X₁₀ is ATQ; and more preferably, X₁₉ is S, X₂₀ is S, X₂₃ is L, X₂₄ is K, X₂₅ is P, and X₃₇ is trans-Hyp, and further preferably, X₂₁ is N, Q, E, S, T, A, G, H, K, or R and/or X₂₂ is N, Q, E, S, NMeAsn, αMeAsn, or NMeAsp.

In some embodiments of the present application, the sequence of the polypeptide comprises any one of sequences of SEQ ID No. 1-SEQ ID No. 25.

In some embodiments of the present application, the sequence of the polypeptide is ASELS TAALG RLADF LRHSS NNLKP ILPPT NVGSNT-trans-Hyp-NH₂ (SEQ ID No. 1).

In some embodiments of the present application, the sequence of the polypeptide is ASQLS TAVLG RLADF LRHSS NNLKP ILPPT NVGSN T-trans-Hyp-NH₂ (SEQ ID No. 2).

In some embodiments of the present application, the sequence of the polypeptide is ASELS TAALG RLADF LRHFT DRDRD ILPPT NVGSN TP-NH₂ (SEQ ID No. 3).

In some embodiments of the present application, the sequence of the polypeptide is ASQLS TAVLG RLADF LRHFT DRDRD ILPPT NVGSN TP-NH₂ (SEQ ID No. 4).

In some embodiments of the present application, the sequence of the polypeptide is ASQLS TAATQ RLADFLRHSS NNLKPILPPT NVGSNT-trans-Hyp-NH₂ (SEQ ID No. 5).

In some embodiments of the present application, the sequence of the polypeptide is ASQLS TAATQ RLADF LRHFT DRDRD ILPPT NVGSN TP-NH₂ (SEQ ID No. 6).

In some embodiments of the present application, the sequence of the polypeptide is ASQLS TAVTQ RLADFLRHSS NNLKPILPPT NVGSNT-trans-Hyp-NH₂ (SEQ ID No. 7).

In some embodiments of the present application, the sequence of the polypeptide is ASQLS TAVTQ RLADF LRHFT DRDRD ILPPT NVGSN TP-NH₂ (SEQ ID No. 8).

In some embodiments of the present application, the sequence of the polypeptide is ASQLS TAVLQ RLADFLRHSS NNLKPILPPT NVGSNT-trans-Hyp-NH₂ (SEQ ID No. 9).

In some embodiments of the present application, the sequence of the polypeptide is ASQLS TAVLQ RLADF LRHFT DRDRD ILPPT NVGSN TP-NH₂ (SEQ ID No. 10).

In some embodiments of the present application, the sequence of the polypeptide is ASQLS TAATQ RLADF LRHSS QQLKP ILPPT NVGSN T-*trans*-Hyp-NH₂ (SEQ ID No. 11).

In some embodiments of the present application, the sequence of the polypeptide is ASQLS TAATQ RLADF LRHSS EELKP ILPPT NVGSN T-*trans*-Hyp-NH₂ (SEQ ID No. 12).

In some embodiments of the present application, the sequence of the polypeptide is ASQLS TAATQ RLADF LRHSS SSLKP ILPPT NVGSN T-*trans*-Hyp-NH₂ (SEQ ID No. 13).

In some embodiments of the present application, the sequence of the polypeptide is ASQLS TAATQ RLADF LRHSS N-NMeAsn-LKP ILPPT NVGSN T-*trans*-Hyp-NH₂ (SEQ ID No. 14).

In some embodiments of the present application, the sequence of the polypeptide is ASQLS TAATQ RLADF LRHSS N-NMeAsp-LKP ILPPT NVGSN T-*trans*-Hyp-NH₂ (SEQ ID No. 15).

In some embodiments of the present application, the sequence of the polypeptide is ASQLS TAATQ RLADF LRHSS QNLKP ILPPT NVGSN T-*trans*-Hyp-NH₂ (SEQ ID No. 16).

In some embodiments of the present application, the sequence of the polypeptide is ASQLS TAATQ RLADF LRHSS ENLKP ILPPT NVGSN T-*trans*-Hyp-NH₂ (SEQ ID No. 17).

In some embodiments of the present application, the sequence of the polypeptide is ASQLS TAATQ RLADF LRHSS SNLKP ILPPT NVGSN T-*trans*-Hyp-NH₂ (SEQ ID No. 18).

In some embodiments of the present application, the sequence of the polypeptide is ASQLS TAATQ RLADF LRHSS TNLKP ILPPT NVGSN T-*trans*-Hyp-NH₂ (SEQ ID No. 19).

In some embodiments of the present application, the sequence of the polypeptide is ASQLS TAATQ RLADF LRHSS ANLKP ILPPT NVGSN T-*trans*-Hyp-NH₂ (SEQ ID No. 20).

In some embodiments of the present application, the sequence of the polypeptide is ASQLS TAATQ RLADF LRHSS GNLKP ILPPT NVGSN T-*trans*-Hyp-NH₂ (SEQ ID No. 21).

In some embodiments of the present application, the sequence of the polypeptide is ASQLS TAATQ RLADF LRHSS HNLKP ILPPT NVGSN T-*trans*-Hyp-NH₂ (SEQ ID No. 22).

In some embodiments of the present application, the sequence of the polypeptide is ASQLS TAATQ RLADF LRHSS KNLKP ILPPT NVGSN T-*trans*-Hyp-NH₂ (SEQ ID No. 23).

In some embodiments of the present application, the sequence of the polypeptide is ASQLS TAATQ RLADF LRHSS RNLKP ILPPT NVGSN T-*trans*-Hyp-NH₂ (SEQ ID No. 24).

In some embodiments of the present application, the sequence of the polypeptide is ASQLS TAATQ RLADF LRHSS N-αMeAsn-LKP ILPPT NVGSN T-*trans*-Hyp-NH₂ (SEQ ID No. 25).

In some embodiments of the present application, the fatty acid-containing side chain is in the form of Z1+Z2+Z3, wherein Z1 is a C16-C22 fatty diacid; Z2 is selected from any one of yGlu, αGlu, βAsp, αAsp, Inp, and Trx, or is absent; Z3 is 0-6 AEEA(s), for example 0, 1, 2, 3, 4, 5, or 6, AEEA(s); and Z1, Z2, and Z3 are linked via amide linkages.

In some embodiments of the present application, Z1 is a C22 fatty diacid.

In some embodiments of the present application, Z1 is a C20 fatty diacid.

In some embodiments of the present application, Z1 is a C19 fatty diacid.

In some embodiments of the present application, Z1 is a C18 fatty diacid.

In some embodiments of the present application, Z1 is a C17 fatty diacid.

In some embodiments of the present application, Z1 is a C16 fatty diacid.

In some embodiments of the present application, Z2 is yGlu.

In some embodiments of the present application, Z2 is αGlu.

In some embodiments of the present application, Z2 is βAsp.

In some embodiments of the present application, Z2 is αAsp.

In some embodiments of the present application, Z2 is Inp.

In some embodiments of the present application, Z2 is Trx.

In some embodiments of the present application, Z2 is absent.

In some embodiments of the present application, Z3 is absent.

In some embodiments of the present application, Z3 is 1 AEEA.

In some embodiments of the present application, Z3 is 2 AEEAs.

In some embodiments of the present application, Z3 is 3 AEEAs.

In some embodiments of the present application, Z3 is 4 AEEAs.

In some embodiments of the present application, Z3 is 5 AEEAs.

In some embodiments of the present application, Z3 is 6 AEEAs.

In some embodiments of the present application, the derivative of the polypeptide is M1, or M2, or M3, or M4, or M5, or M6, or M7, or M8, or M9, or M10, or M11, or M12, or M13, or M14, or M15, or M16, or M17, or M18, or M19, or M20, or M21, or M22, or M23, or M24, or M25, or M26, or M27, or M28, or M29, or M30, or M31, or M32, or M33, or M34, or M35, or M36, or M37, or M38, or M39, or M40, or M41, or M42, or M43, or M44, or M45, or M46, or M47, or M48, or M49, or M50, or M51, or M52, or M53, or M54, or M55.

Provided in the present application is a polypeptide comprising a polypeptide sequence described above.

In some embodiments of the present application, the polypeptide sequence comprised in the polypeptide is any one of SEQ ID No. 1-SEQ ID No. 25.

Provided in the present application is a pharmaceutical composition comprising the polypeptide described above, or the derivative or a pharmaceutically acceptable salt thereof described above, and a pharmaceutically acceptable excipient.

In some embodiments of the present application, the pharmaceutical composition further comprises another one or more active pharmaceutical ingredients. The pharmacologically active ingredients comprise a body weight regulator, an anti-obesity agent, a lipid metabolism regulator, a glycemic regulator, a hypertension therapeutic, a cardiovascular regulator, and a regulator for a cerebral system disease, a psychiatric disease, or the nervous system. Preferably, the pharmacologically active substances are selected from GLP-1 receptor agonists including but not limited to GLP-1, GLP-1 analogs and GLP-1 derivatives, GIP receptor agonists including but not limited to GIP, GIP analogs and GIP derivatives, and insulin receptor agonists including but not limited to insulin, insulin analogs and insulin derivatives.

The polypeptide described above, the derivative or a pharmaceutically acceptable salt thereof described above, or the composition is for use in the manufacture of a medicament for preventing and/or treating an amylin receptor related metabolic disease or a lipid metabolism disorder.

The polypeptide described above, the derivative or a pharmaceutically acceptable salt thereof described above, or the composition is for use in the manufacture of a medicament for preventing and/or treating overweight and/or obesity and/or type I or type II diabetes and/or osteoporosis and/or neuropathic pain.

The polypeptide described above, the derivative or a pharmaceutically acceptable salt thereof described above, or the composition is for use in the manufacture of a medicament for reducing food intake. The polypeptide described above, the derivative or a pharmaceutically acceptable salt thereof described above, or the composition is for use in reducing food intake.

Provided in the present application is a method of preventing and/or treating an amylin receptor related metabolic disease or a lipid metabolism disorder, comprising administering to a subject a prophylactically or therapeutically effective amount of the polypeptide described above, the derivative or a pharmaceutically acceptable salt thereof described above, or the pharmaceutical composition described above.

Provided in the present application is a method of preventing and/or treating overweight and/or obesity and/or type I or type II diabetes and/or osteoporosis and/or neuropathic pain, comprising administering to a subject a prophylactically or therapeutically effective amount of the polypeptide described above, the derivative or a pharmaceutically acceptable salt thereof described above, or the pharmaceutical composition described above.

Provided in the present application is a method of reducing food intake, comprising administering to a subject an effective amount of the polypeptide described above, the derivative or a pharmaceutically acceptable salt thereof described above, or the pharmaceutical composition described above.

The polypeptides and their derivatives or compositions of the present application have remarkably improved advantages or positive effects in terms of pharmaceutical activity, molecular stability, and bioavailability *in vivo* as compared with those in the prior art at an equivalent dosage. Besides, as opposed to traditional amylin analogs or derivatives, the polypeptides and their derivatives described in the present application contain no disulfide bond but surprisingly exhibit an unexpectedly high stability, show a decreased tendency for fibrillation, and still have a relatively high stability even when formulated under a neutral or non-acidic pH. Further, the polypeptides or their derivatives of the present application have desirable pharmacokinetic effects and therefore are not required to be injected as frequently as those known amylin derivatives.

### BRIEF DESCRIPTION OF THE DRAWINGS

FIG. 1 shows the results of a heat accelerated assay for the polypeptide derivative M6 in Example 3;
FIG. 2A shows the effect of the polypeptide derivatives on weight loss in a SD rat model over time in Example 4;
FIG. 2B shows the effect of the polypeptide derivatives on food intake in a SD rat model over time in Example 4;
FIGs. 3A and 3C show the effect of the polypeptide derivatives at different dosages on weight loss in a SD rat model over time in Example 5;
FIGs. 3B and 3D show the effect of the polypeptide derivatives at different dosages on food intake in a SD rat model over time in Example 5;
FIG. 4A shows the effect of the polypeptide derivatives at different dosages on weight loss in a DIO SD rat model over time in Example 6;
FIG. 4B shows the effect of the polypeptide derivatives at different dosages on food intake in a DIO SD rat model over time in Example 6;
FIGs. 5A-5B show the effect of the polypeptide derivatives on weight loss in a SD rat model over time in Example 7;
FIGs. 5C-5D show the effect of the polypeptide derivatives on food intake in a SD rat model over time in Example 7; and
FIG. 6 is a plot showing the concentration of different molecules in plasma over time in Example 8.

### DETAILED DESCRIPTION

The present application is further described below with examples. It is to be understood that the examples are for the purpose of further illustrating and setting forth the present application only, and are not intended to limit the present application.

Terms regarding technology and science in this specification have the same meaning as usually understood by those skilled in the art, unless otherwise defined. Although a description of methods and materials is made hereinbelow, methods and materials similar or identical to those described herein may be used in assays or practical applications. Where there is a contradiction, definitions included in this specification take precedence. Additionally, materials, methods, and examples are only illustrative and not limiting. Further description of the present application is presented as below in conjunction with specific examples, which is not for limiting the scope of the present application.

The terms "polypeptide", "peptide", and "protein" are used interchangeably herein and refer to an amino acid polymer of any length. The polymer can be linear or branched and may comprise naturally occurring amino acids among which non-naturally occurring ones may be interspersed.

The "derivative" described herein refers to a product resulting from a modification of a functional group (e.g., an amino acid residue) in a biomacromolecule (e.g., a polypeptide or protein) with certain compounds or molecules. The modification includes but is not limited to acylation, amidation, esterification, and thioesterification.

A pharmaceutical composition herein may be used for the treatment of diseases or for cell culture assays *in vitro.* When used for the treatment of diseases, the term "pharmaceutical composition" generally refers to a unit dosage form and may be prepared by any of the methods well known in the field of pharmaceutics. The methods used include a step of blending the active ingredients with excipients that are one or more auxiliary ingredients. Typically, a composition is prepared by uniformly and adequately blending active compounds with a liquid excipient, a fine solid excipient or both.

Being "pharmaceutically acceptable" herein refers to an attribute of a substance or composition that has to be chemically and/or toxicologically compatible with other ingredients contained in the formulation and/or mammals treated thereby.

The term "a pharmaceutically acceptable excipient" herein may include any solvent, solid excipient, diluent or other liquid excipients or the like suitable for particular target dosage forms. Use of any conventional excipients is also within the scope contemplated in the present application, except to the extent that they are incompatible with the compounds of the present application, for example, result in any undesirable biological effects or interact with the any other components of the pharmaceutically acceptable composition in a deleterious way.

Treatment herein refers to the attainment of a desired pharmacological and/or physiological effect. The effect can be prophylactic in terms of completely or partially preventing a disease or a symptom thereof, and/or can be therapeutic in terms of completely or partially curing a disease and/or an adverse effect attributable to the disease. The "treatment" used herein encompasses diseases of mammals, particular human, and comprises: (a) preventing occurrence of a disease or condition in an individual susceptible to but not diagnosed with the disease; (b) inhibiting a disease, for example, impeding the development of the disease; or (c) alleviating a disease, for example, relieving symptoms related to the disease. The "treatment" used herein encompasses any administration of a medicament or compound to an individual to treat, cure, alleviate, ameliorate, palliate or inhibit a disease of the individual, including but not limited to giving a medicament comprising the compound described herein to an individual in need thereof.

"Prevention" herein refers to lower the possibility of occurrence (or recurrence) of a disease, condition, or disorder or related symptoms (e.g., cancer).

An "amylin receptor related metabolic disease" herein is a disease where a subject's symptoms can be ameliorated by modulating activation of an amylin receptor to improve the situation of downstream cellular pathways, including hyperlipidemia, atherosclerosis, hypertension, coronary heart disease, myocardial infarction, cerebral thrombosis, cerebral hemorrhage, cerebral embolism, obesity, fatty liver, cirrhosis, diabetes, and diseases usually accompanying lipid metabolism disorders such as osteoporosis, cognitive disorders, neurodegenerative diseases (e.g., Parkinson's syndrome and Alzheimer's diseases), and gastrointestinal diseases, for example, an inflammatory bowel disease, dystrophy, or digestive tract ulcer.

In some embodiments of the present application, the polypeptide is coupled to fatty acid by means of an amide linkage in the polypeptide derivative.

In some embodiments of the present application, the fatty acid is C1-C25 fatty acid, for example, formic acid, acetic acid, C3 fatty acid, C4 fatty acid, C5 fatty acid, C6 fatty acid, C7 fatty acid, C8 fatty acid, C9 fatty acid, C10 fatty acid, C11 fatty acid, C12 fatty acid, C13 fatty acid, C14 fatty acid, C15 fatty acid, C16 fatty acid, C17 fatty acid, C18 fatty acid, C19 fatty acid, C20 fatty acid, C21 fatty acid, C22 fatty acid, C23 fatty acid, C24 fatty acid or C25 fatty acid.

The C1-C25 fatty acid in the present application may comprise linear fatty acids and branched fatty acids.

The C1-C25 fatty acid in the present application may comprise saturated fatty acids and unsaturated fatty acids.

In some embodiments of the present application, the fatty acid is one or more fatty acids selected from the group consisting of octanoic acid, nonanoic acid, decanoic acid, undecanoic acid, lauric acid, tridecanoic acid, myristic acid, pentadecanoic acid, palmitic acid, heptadecanoic acid, stearic acid, nonadecanoic acid, arachidic acid, heneicosanoic acid, docosanoic acid, tricosanoic acid, tetracosanoic acid, palmitoleic acid, oleic acid, linoleic acid, ricinoleic acid, isocitric acid, eicosapentaenoic acid, docosahexaenoic acid, 1,8-octanedioic acid, 1,7-heptanedicarboxylic acid, 1,10-decanedioic acid, undecanedioic acid, dodecanedioic acid, tridecanedioic acid, tetradecanedioic acid, pentadecanedioic acid, hexadecanedioic acid, heptadecanedioic acid, octadecanedioic acid, nonadecanedioic acid, eicosanedioic acid, heneicosanedioic acid, docosanedioic acid, and tricosanedioic acid.

The fatty acid moiety of the present application may be linked to the polypeptide described above via a linker selected from one or more of -yGlu-, -αGlu-, -βAsp-, -αAsp-, -Inp-, -Trx-, 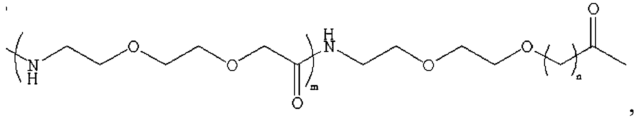 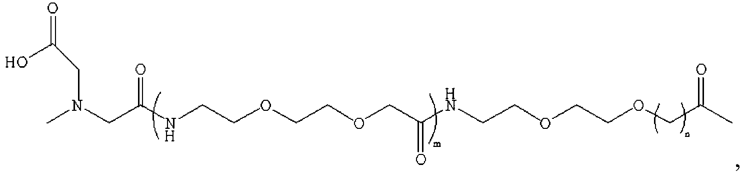 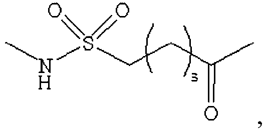 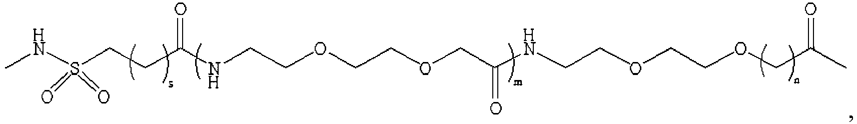 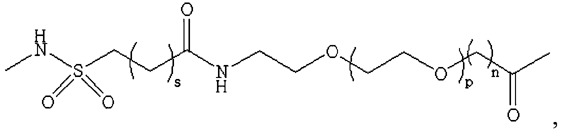 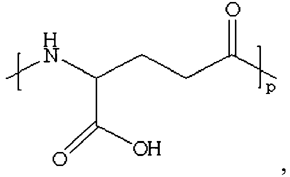 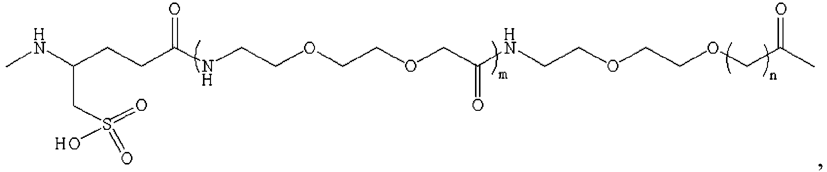 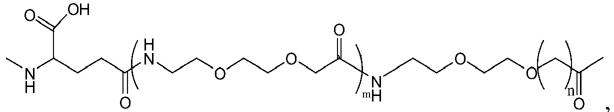 or 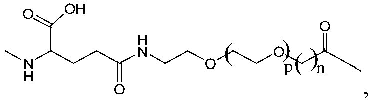 wherein m is 0, 1, 2, or 3; n is 1, 2, or 3; s is any integer of 0-6; and p is any integer of 1-8.

In the present application, the term "a pharmaceutically acceptable salt" refers to a salt that retains biological effectiveness and properties of the derivative of the polypeptide in the present application and is generally not biologically or otherwise undesired. In many cases, the polypeptide derivative of the present application may form an acid and/or base addition salt due to the presence of an amino and/or carboxyl group or a similar group.

Inorganic and organic acids may be used to form a pharmaceutically acceptable acid addition salt, for example, acetate, aspartate, benzoate, benzenesulfonate, bromide/hydrobromide, bicarbonate/carbonate, bisulfate/sulfate, camphorsulfonate, chloride/hydrochloride, chlortheophyllonate, citrate, ethanedisulfonate, fumarate, glucoheptonate, gluconate, glucuronate, hippurate, hydroiodide/iodide, isethionate, lactate, lactobionate, laurylsulfate, malate, maleate, malonate, mandelate, mesylate, methylsulfate, naphthoate, naphthalenesulfonate, nicotinate, nitrate, octadecanoate, oleate, oxalate, palmitate, pamoate, phosphate/hydrophosphate/dihydrogen phosphate, polygalacturonate, propionate, stearate, succinate, sulfosalicylate, tartrate, toluenesulfonate and trifluoroacetate.

Inorganic acids from which salts may be derived include for example hydrochloric acid, hydrobromic acid, sulfuric acid, nitric acid, and phosphoric acid.

Organic acids from which salts may be derived include for example acetic acid, propionic acid, hydroxyacetic acid, oxalic acid, maleic acid, malonic acid, succinic acid, fumaric acid, tartaric acid, citric acid, benzoic acid, mandelic acid, methanesulfonic acid, ethanesulfonic acid, toluenesulfonic acid, and sulfosalicylic acid.

A pharmaceutically acceptable base addition salt may be formed with inorganic and organic base.

Inorganic bases from which salts may be derived include for example ammonium salts and metals of Group I to Group XII in the periodic table. In certain embodiments, the salts are derived from sodium, potassium, ammonium, calcium, magnesium, iron, silver, zinc and copper; and especially appropriate salts include ammonium, potassium, sodium, calcium, and magnesium salts.

Organic bases from which salts may be derived may include for example primary, secondary, and tertiary amines, substituted amine including naturally occurring substituted amine, cyclic amine, and basic ion exchange resin,. Certain organic bases include isopropylamine, choline salts, diethanol amine, diethylamine, lysine, meglumine, piperazine, and tromethamine.

The pharmaceutically acceptable salts of the present application may be synthesized from the parent compound or a basic or acidic moiety thereof by conventional chemical methods. Typically, these salts may be prepared by reacting those compounds in the form of free acids with a stoichiometric amount of appropriate bases (e.g., hydroxide, carbonate, bicarbonate, etc., of Na or K) or by reacting those compounds in the form of free bases with a stoichiometric amount of appropriate acids. These reactions are usually conducted in water, an organic solvent, or a mixture of both. Generally, it is desirable to use nonaqueous media such as ether, ethyl acetate, ethanol, isopropanol, or acetonitrile if possible.

The present application provides a pharmaceutical composition comprising the polypeptide described above or the derivative or a pharmaceutically acceptable salt thereof described above, and a pharmaceutically acceptable excipient. In preferred embodiments, the pharmaceutical composition may further comprise a buffering system, preservative, surfactant, chelator, stabilizer, and surface active agent. In some embodiments, the buffering agent is selected from sodium acetate, sodium carbonate, citrate, glycylglycine, histidine, glycine, lysine, arginine, sodium dihydrogen phosphate, disodium hydrogen phosphate, sodium phosphate, tri(hydroxymethyl)aminomethane, N-di(hydroxyethyl)glycine, tricine, malic acid, lactic acid, succinate, maleic acid, fumaric acid, tartaric acid, aspartic acid or a mixture thereof. Each of these particular buffering agents or a combination thereof constitutes an alternative to the present application. In some embodiments, the medicament or preparation described in the present application is a water-containing medicament or preparation, which for example may typically be a solution or suspension. In some other specific embodiments of the present application, the medicament or preparation is a lyophilized preparation to which a solvent and/or diluent is added prior to use.

The present application provides use of the polypeptide described above or the derivative or a pharmaceutically acceptable salt thereof described above in the manufacture of a medicament for preventing and/or treating an amylin receptor related metabolic disease or a lipid metabolism disorder.

The present application provides use of the polypeptide described above or the derivative or a pharmaceutically acceptable salt thereof described above in the manufacture of a medicament for preventing and/or treating overweight and/or obesity and/or type I or type II diabetes and/or osteoporosis and/or neuropathic pain.

The present application provides use of the polypeptide described above or the derivative or a pharmaceutically acceptable salt thereof described above in the manufacture of a medicament for reducing food intake.

The present application provides use of the polypeptide described above or the derivative or a pharmaceutically acceptable salt thereof described above in reducing food intake.

The present application also relates to a method of preventing and/or treating an amylin receptor related disease or a lipid metabolism disorder related disease, comprising administering to a subject a prophylactically or therapeutically effective amount of the polypeptide described above, the derivative or a pharmaceutically acceptable salt thereof described above, or the pharmaceutical composition described above.

The present application provides a method of preventing and/or treating overweight and/or obesity and/or type I or type II diabetes and/or osteoporosis and/or neuropathic pain, comprising administering to a subject a prophylactically or therapeutically effective amount of the polypeptide described above, the derivative or a pharmaceutically acceptable salt thereof described above, or the pharmaceutical composition described above.

The present application provides a method of reducing food intake, comprising administering to a subject an effective amount of the polypeptide described above, the derivative or a pharmaceutically acceptable salt thereof described above, or the pharmaceutical composition as described above.

Included in some embodiments of the present application is administration, to a subject in need thereof, of the polypeptide described above, the derivative or a pharmaceutically acceptable salt thereof described above, or the pharmaceutical composition.

In some embodiments of the present application, the polypeptide described, the derivative or a pharmaceutically acceptable salt thereof described above, or the pharmaceutical composition is used in combination with other drugs, pharmaceutical compounds or compositions.

### Abbreviations

Corresponding names or structures of some abbreviations used in the examples of the present application are as follows:

| **Abbr.** | **Structural formula** |
|---|---|
| Trx | 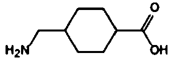 |
| Inp | 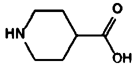 |
| AEEA | 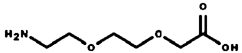 |

AA: Amino Acid
Boc: t-Butyloxy carbonyl
DCM: dichloromethane
DMF: N,N-Dimethyl formamide
DIEA: N,N-Diisopropylethylamine
EDT: 1,2-Ethanedithiol
Fmoc: 9-fluorenylmethyloxycarbonyl
OtBu: t-butyl ester
Pbf: 2,2,4,6,7-Pentamethyldihydrobenzofuran-5-sulfonyl
Pip: Piperidine
TBT: O-(Benzotriazol-1-yl)-N,N,N',N',-tetramethyluronium Tetrafluoroborate
tBu: tertiary butyl
TFA: Trifluoroacetic acid
TIS: Triisopropylsilane
Trt: Triphenylmethyl
αGlu: α-glutamic acid
yGlu: γ-glutamic acid
αAsp: α-aspartic acid
βAsp: β-aspartic acid
αMeAsn: α-methylated asparagine
NMeAsn: N-methylated asparagine
NMeAsp: N-methylated aspartic acid

### Example 1 Preparation of Polypeptide Derivatives

Polypeptides and derivatives thereof prepared in the present application are shown in Table 2.

The polypeptides were synthesized by solid phase organic synthesis, specifically, solid-phase peptide synthesis (SPPS) with Fmoc-protected amino acids, and then cleaved, oxidized, and purified to yield target products in Table 2 sequentially designated as Cagrilintide and M1-M55.

Taking the compound Cagrilintide as an example, the synthesis process was as below:

### 1.1 Solid-phase synthesis

Using Fmoc-Linker MBHA Resin S=0.32mmol/g and employing Fmoc/tBu processing, amino acids were linked by sequential condensation from the C terminus to the N terminus (from right to left) as per the sequence of the aforesaid peptide in accordance with the method provided in Table 1:

**Table 1. Listing of polypeptide synthesis procedures**

| **No.** | **Solvent** | **Number of times** | **duration (min/time)** |
|---|---|---|---|
| 1 | 20% Pip/DMF | 1 | 10 |
| 2 | 20% Pip/DMF | 1 | 5 |
| 3 | DMF | 5 | 1 |
| 4 | Sampling for colorimetric testing | | |
| 5 | AA/DIEA/TBTU | 1 | 60 |
| 6 | DMF | 4 | 1 |
| 7 | Sampling for colorimetric testing | | |
| Notes | Repeating 1-7 till the end of the linear peptide synthesis | | |

The following amino acids were coupled in order:
A-01 Fmoc-Pro-OH, A-02 Fmoc-Thr(tBu)-OH, A-03 Fmoc-Asn(Trt)-OH, A-04 Fmoc-Ser(tBu)-OH, A-05 Fmoc-Gly-OH, A-06 Fmoc-Val-OH, A-07 Fmoc-Asn(Trt)-OH, A-08 Fmoc-Thr(tBu)-OH, A-09 Fmoc-Pro-OH, A-10 Fmoc-Pro-OH, A-11 Fmoc-Leu-OH, A-12 Fmoc-Ile-OH, A-13 Fmoc-Pro-OH, A-14 Fmoc-Gly-OH, A-15 Fmoc-Phe-OH, A-16 Fmoc-Asn(Trt)-OH, A-17 Fmoc-Asn(Trt)-OH, A-18 Fmoc-Ser(tBu)-OH, A-19 Fmoc-Ser(tBu)-OH, A-20 Fmoc-His(Trt)-OH, A-21 Fmoc-Arg(Pbf)-OH, A-22 Fmoc-Leu-OH, A-23 Fmoc-Phe-OH, A-24 Fmoc-Glu(OtBu)-OH, A-25 Fmoc-Ala-OH, A-26 Fmoc-Leu-OH, A-27 Fmoc-Arg(Pbf)-OH, A-28 Fmoc-Gln(Trt)-OH, A-29 Fmoc-Thr(tBu)-OH, A-30 Fmoc-Ala-OH, A-31 Fmoc-Cys(Trt)-OH, A-32 Fmoc-Thr(tBu)-OH, A-33 Fmoc-Ala-OH, A-34 Fmoc-Thr(tBu)-OH, A-35 Fmoc-Asn(Trt)-OH, A-36 Fmoc-Cys(Trt)-OH, A-37 Fmoc-Lys(Boc)-OH, A-38 Fmoc-Glu-OtBu, and A-39 C20 diacid.

Finally a polypeptide derivative-on-resin was formed.

The polypeptide derivative-on-resin was washed, transferred out, and dried to a constant weight for cleavage.

### 1.2 Cleavage

Formulation of a cleavage reagent: the cleavage reagent was TFA:H₂O:EDT:TIS = 95:1:2:2, the amount of which was a volume of 10 mL±2 mL per 1 g of the polypeptide derivative-on-resin. H₂O, TFA, EDT, and TIS composing the required cleavage reagent were sequentially added to a cleavage reaction flask and were controlled at a temperature of 0-10°C. The cleavage reagent was added to the resin under stirring, and after the temperature of the system became stable, the reactants were stirred for another 2.5 hours under a controlled temperature of 25-30°C. The cleavage solution was filtered, and allowed for precipitation using glacial diethyl ether of a 5x volume of the liquid. The precipitates were filtered, washed 3 times with glacial diethyl ether of a 3x volume of the liquid, and then dried under reduced pressure at room temperature to yield a solid crude.

### 1.3 Oxidation

The crude was finely grounded, and was slowly added, with stirring, to purified water, while an aqueous solution of acetonitrile was added dropwise. After the crude was added and completely dissolved, a methanol solution of iodine was added and stirred for half an hour.

### 1.4 Purification and lyophilization

The oxidated liquid as described above was filtered with a 0.45 µm microporous filtering membrane. The crude was separated and purified using a column prepared by C-18 column packing material with a suitable gradient at ambient temperature, and then the target product was collected, detected and analyzed, and sorted. A purity ≥90% was required. Substandard target products were collected, and separated and purified again with a suitable gradient, to achieve qualified liquid peaks. The qualified liquid samples as mentioned above were lyophilized under reduced pressure to yield lyophilized powder of the refined polypeptide derivative.

Polypeptide derivatives prepared in the present application are shown as M1-M55 in Table 2 and are distinguished from each other by the amino acid sequence or the fatty acid side chain.

**Table 2. Summary of designed polypeptides and derivatives thereof with modification at the N terminus**

| ID | Amino acid sequence | Fatty acid-containing side chain |
|---|---|---|
| Cagrilinti de | KCNTATCATQ RLAEFLRHSS NNFGPILPPT NVGSNTP-NH₂ | C20 diacid + yGlu |
| M1 | ASELS TAALG RLADF LRHSS NNLKP ILPPT NVGSN T-*trans*-Hyp-NH₂ (SEQ ID No. 1) | C20 diacid+γGlu |
| M2 | ASQLS TAVLG RLADF LRHSS NNLKP ILPPT NVGSN T-*trans*-Hyp-NH₂ (SEQ ID No. 2) | C20 diacid+yGlu |
| M3 | ASELS TAALG RLADF LRHFT DRDRD ILPPT NVGSN TP-NH₂ (SEQ ID No. 3) | C20 diacid+γGlu |
| M4 | ASQLS TAVLG RLADF LRHFT DRDRD ILPPT NVGSN TP-NH₂ (SEQ ID No. 4) | C20 diacid+γGlu |
| M5 | ASQLS TAATQ RLADFLRHSS NNLKPILPPT NVGSNT-trans-Hyp-NH₂ (SEQ ID No. 5) | C20 diacid+yGlu |
| M6 | ASQLS TAATQ RLADF LRHFT DRDRD ILPPT NVGSN TP-NH₂ (SEQ ID No. 6) | C20 diacid+γGlu |
| M7 | ASQLS TAVTQ RLADFLRHSS NNLKPILPPT NVGSNT-trans-Hyp-NH₂ (SEQ ID No. 7) | C20 diacid+γGlu |
| M8 | ASQLS TAVTQ RLADF LRHFT DRDRD ILPPT NVGSN TP-NH₂ (SEQ ID No. 8) | C20 diacid+γGlu |
| M9 | ASQLS TAVLQ RLADFLRHSS NNLKPILPPT NVGSNT-trans-Hyp-NH₂ (SEQ ID No. 9) | C20 diacid+yGlu |
| M10 | ASQLS TAVLQ RLADF LRHFT DRDRD ILPPT NVGSN TP-NH₂ (SEQ ID No. 10) | C20 diacid+γGlu |
| M11 | ASQLS TAATQ RLADF LRHFT DRDRD ILPPT NVGSN TP-NH₂ | C20 diacid + αGlu |
| M12 | ASQLS TAATQ RLADF LRHFT DRDRD ILPPT NVGSN TP-NH₂ | C20 diacid + βAsp |
| M13 | ASQLS TAATQ RLADF LRHFT DRDRD ILPPT NVGSN TP-NH₂ | C20 diacid + αAsp |
| M14 | ASQLS TAATQ RLADF LRHFT DRDRD ILPPT NVGSN TP-NH₂ | C20 diacid + Trx |
| M15 | ASQLS TAATQ RLADF LRHFT DRDRD ILPPT NVGSN TP-NH₂ | C20 diacid + Inp |
| M16 | ASQLS TAATQ RLADF LRHFT DRDRD ILPPT NVGSN TP-NH₂ | **C20 diacid** + γGlu+2*AEEAs |
| M17 | ASQLS TAATQ RLADF LRHFT DRDRD ILPPT NVGSN TP-NH₂ | C20 diacid + αGlu+2*AEEAs |
| M18 | ASQLS TAATQ RLADF LRHFT DRDRD ILPPT NVGSN TP-NH₂ | C20 diacid + βAsp+2*AEEAs |
| M19 | ASQLS TAATQ RLADF LRHFT DRDRD ILPPT NVGSN TP-NH₂ | C20 diacid + αAsp+2*AEEAs |
| M20 | ASQLS TAATQ RLADF LRHFT DRDRD ILPPT NVGSN TP-NH₂ | C20 diacid + Trx+2*AEEAs |
| M21 | ASQLS TAATQ RLADF LRHFT DRDRD ILPPT NVGSN TP-NH₂ | C20 diacid + Inp+2*AEEAs |
| M22 | ASQLS TAATQ RLADF LRHFT DRDRD ILPPT NVGSN TP-NH₂ | C18 diacid + γGlu |
| M23 | ASQLS TAATQ RLADF LRHFT DRDRD ILPPT NVGSN TP-NH₂ | C16 diacid + γGlu |
| M24 | ASQLS TAATQ RLADF LRHFT DRDRD ILPPT NVGSN TP-NH₂ | C20 diacid + γGlu+4*AEEAs |
| M25 | ASQLS TAATQ RLADF LRHFT DRDRD ILPPT NVGSN TP-NH₂ | C20 diacid + yGlu+6*AEEAs |
| M26 | ASQLS TAATQ RLADFLRHSS NNLKPILPPT NVGSNT-trans-Hyp-NH₂ | C20 diacid + αGlu |
| M27 | ASQLS TAATQ RLADFLRHSS NNLKPILPPT NVGSNT-trans-Hyp-NH₂ | C20 diacid + βAsp |
| M28 | ASQLS TAATQ RLADFLRHSS NNLKPILPPT NVGSNT-trans-Hyp-NH₂ | C20 diacid + αAsp |
| M29 | ASQLS TAATQ RLADFLRHSS NNLKPILPPT NVGSNT-trans-Hyp-NH₂ | C20 diacid + Trx |
| M30 | ASQLS TAATQ RLADFLRHSS NNLKPILPPT NVGSNT-trans-Hyp-NH₂ | C20 diacid + Inp |
| M31 | ASQLS TAATQ RLADFLRHSS NNLKPILPPT NVGSNT-trans-Hyp-NH₂ | C20 diacid + γGlu+2*AEEAs |
| M32 | ASQLS TAATQ RLADFLRHSS NNLKPILPPT NVGSNT-trans-Hyp-NH₂ | C20 diacid + αGlu+2*AEEAs |
| M33 | ASQLS TAATQ RLADFLRHSS NNLKPILPPT NVGSNT-trans-Hyp-NH₂ | C20 diacid + βAsp+2*AEEAs |
| M34 | ASQLS TAATQ RLADFLRHSS NNLKPILPPT NVGSNT-trans-Hyp-NH₂ | C20 diacid + αAsp+2*AEEAs |
| M35 | ASQLS TAATQ RLADFLRHSS NNLKPILPPT NVGSNT-trans-Hyp-NH₂ | C20 diacid + Trx+2*AEEAs |
| M36 | ASQLS TAATQ RLADFLRHSS NNLKPILPPT NVGSNT-trans-Hyp-NH₂ | C20 diacid + Inp+2*AEEAs |
| M37 | ASQLS TAATQ RLADFLRHSS NNLKPILPPT NVGSNT-trans-Hyp-NH₂ | C18 diacid + γGlu |
| M38 | ASQLS TAATQ RLADFLRHSS NNLKPILPPT NVGSNT-trans-Hyp-NH₂ | C16 diacid + γGlu |
| M39 | ASQLS TAATQ RLADFLRHSS NNLKPILPPT NVGSNT-trans-Hyp-NH₂ | C20 diacid + γGlu+4*AEEAs |
| M40 | ASQLS TAATQ RLADFLRHSS NNLKPILPPT NVGSNT-trans-Hyp-NH₂ | C20 diacid + γGlu+6*AEEAs |
| M41 | ASQLS TAATQ RLADF LRHSS QQLKP ILPPT NVGSN T-*trans*-Hyp-NH₂(SEQ ID No. 11) | C20 diacid+γGlu |
| M42 | ASQLS TAATQ RLADF LRHSS EELKP ILPPT NVGSN T-*trans*-Hyp-NH₂(SEQ ID No. 12) | C20 diacid+γGlu |
| M43 | ASQLS TAATQ RLADF LRHSS SSLKP ILPPT NVGSN T-*trans*-Hyp-NH₂(SEQ ID No. 13) | C20 diacid+γGlu |
| M44 | ASQLS TAATQ RLADF LRHSS N-NMeAsn-LKP ILPPT NVGSN T-*trans*-Hyp-NH₂(SEQ ID No. 14) | C20 diacid+γGlu |
| M45 | ASQLS TAATQ RLADF LRHSS N-NMeAsp-LKP ILPPT NVGSN T-*trans*-Hyp-NH₂(SEQ ID No. 15) | C20 diacid+γGlu |
| M46 | ASQLS TAATQ RLADF LRHSS QNLKP ILPPT NVGSN T-*trans*-Hyp-NH₂(SEQ ID No. 16) | C20 diacid+γGlu |
| M47 | ASQLS TAATQ RLADF LRHSS ENLKP ILPPT NVGSN T-*trans*-Hyp-NH₂(SEQ ID No. 17) | C20 diacid+γGlu |
| M48 | ASQLS TAATQ RLADF LRHSS SNLKP ILPPT NVGSN T-*trans*-Hyp-NH₂(SEQ ID No. 18) | C20 diacid+γGlu |
| M49 | ASQLS TAATQ RLADF LRHSS TNLKP ILPPT NVGSN T-*trans*-Hyp-NH₂(SEQ ID No. 19) | C20 diacid+γGlu |
| M50 | ASQLS TAATQ RLADF LRHSS ANLKP ILPPT NVGSN T-*trans*-Hyp-NH₂(SEQ ID No. 20) | C20 diacid+γGlu |
| M51 | ASQLS TAATQ RLADF LRHSS GNLKP ILPPT NVGSN T-*trans*-Hyp-NH₂(SEQ ID No. 21) | C20 diacid+γGlu |
| M52 | ASQLS TAATQ RLADF LRHSS HNLKP ILPPT NVGSN T-*trans*-Hyp-NH₂(SEQ ID No. 22) | C20 diacid+γGlu |
| M53 | ASQLS TAATQ RLADF LRHSS KNLKP ILPPT NVGSN T-*trans*-Hyp-NH₂(SEQ ID No. 23) | C20 diacid+γGlu |
| M54 | ASQLS TAATQ RLADF LRHSS RNLKP ILPPT NVGSN T-*trans*-Hyp-NH₂(SEQ ID No. 24) | C20 diacid+γGlu |
| M55 | ASQLS TAATQ RLADF LRHSS N-αMeAsn-LKP ILPPT NVGSN T-trans-Hvp-NH₂(SEQ ID No. 25) | C20 diacid+γGlu |

M1-M55 represent derivatives resulting from modifications of amino acid sequences with fatty acid-containing side chains in the present application, for example those derived from SEQ ID No. 1-SEQ ID No. 10 upon modifications with the corresponding fatty acid-containing side chains in Table 2. For instance, M1 represents the derivative M1 offered by modifying SEQ ID No. 1 with C20 diacid+γGlu. The fatty acid-containing side chains are in the form of Z1+Z2+Z3, wherein Z1 is a C16-C22 fatty diacid; Z2 is selected from any one of γGlu, αGlu, βAsp, αAsp, Inp, and Trx, or is absent; Z3 is 0-6, for example 0, 1, 2, 3, 4, 5, or 6, AEEA(s); and Z1, Z2, and Z3 are linked via amide linkages. The C16-C22 fatty diacid moiety is located at the outer side of the fatty acid-containing side chains for modification relative to the amino acid sequence, and thus is positioned at the distal end of the linkage of the side chain d to the amino acid sequence.

### Example 2 Testing for Activity of the Derivatives in Cells

The objective of this assay is to test the potency of derivatives of polypeptides of the present application on human amylin receptors *in vitro* using a luciferase assay.

### 2.1 Construction of an amylin receptor/CRE-luc cell line

Using the standard protocol, CHO-K1/Ga15/AMY3 cells (purchased from GenScript, with the calcitonin receptor and the receptor activity modifying peptide (RAMP) constructed already) were transfected with a plasmid containing a luciferase expression cassette driven by multiple copies of cAMP response elements (CREs). The cells were cultured in an F12 medium containing 200 µg/mL Zeocin, 2 µg/mL puromycin, 100 µg/mL hygromycin, and 400 µg/mL G418 to afford a stably transfected amylin receptor/CRE-luc cell line.

### 2.2 Luciferase assay of amylin

The lyophilized powder from Example 1 was dissolved in 20 mM phosphate buffer solution with a pH of 7.0, and diluted in a growth medium (F12 culture medium containing 10% FBS) to give a derivative sample with an initial concentration of 10 nM, which was then subjected to a 5-fold serial dilution in the growth medium to obtain seven samples with concentrations between 2nM and 0.4nM. In each well of a white 96-well plate, 50 µL of the tested sample solution was added with a corresponding concentration.

The stably transfected amylin receptor/CRE-luc CHO cells were resuspended in the growth medium at a certain density, and 50 µL of the cell suspension was added at a density of about 20000 cells per well to the white 96-well plate containing the tested sample solution. After incubation of 24 hours at 37°C and 5% CO₂, 100 µL of a Luciferase substrate was added to each well, incubated for 3 minutes, and finally was tested for luminescence on SpectraMax L (Molecular Devices) with the software SoftMax Pro 7.0.3 GxP. A standard curve was plotted using the fluorescent intensities for calculation of EC50, and the variability among different polypeptide derivatives was summarized by calculating the relative activity (the EC50 ratio of Cagrilintide to the compound), wherein the relative activity of Cagrilintide was 100%. Results were shown in the following table.

**Table 3. Results for activity against amylin receptors in cells**

| ID | EC50/nM | Relative activity | ID | EC50/nM | Relative activity |
|---|---|---|---|---|---|
| M1 | 0.053 | 49% | M28 | 0.039 | 128% |
| M2 | 0.038 | 68% | M29 | 0.122 | 412% |
| M3 | 0.081 | 32% | M30 | 0.039 | 130% |
| M4 | 0.081 | 32% | M31 | 0.168 | 30% |
| M5 | 0.031 | 84% | M32 | 0.115 | 44% |
| M6 | 0.042 | 62% | M33 | 0.145 | 37% |
| M7 | 0.066 | 39% | M34 | 0.132 | 40% |
| M8 | 0.049 | 53% | M35 | 0.043 | 124% |
| M9 | 0.026 | 100% | M36 | 0.059 | 91% |
| M11 | 0.004 | 110% | M37 | 0.030 | 184% |
| M12 | 0.034 | 78% | M38 | 0.030 | 181% |
| M13 | 0.034 | 78% | M41 | 0.08 | 113% |
| M14 | 0.018 | 136% | M42 | 0.28 | 32% |
| M15 | 0.030 | 81% | M43 | 0.05 | 180% |
| M16 | 0.042 | 58% | M44 | 0.1 | 130% |
| M17 | 0.026 | 94% | M45 | 0.07 | 129% |
| M18 | 0.026 | 110% | M46 | 0.06 | 150% |
| M19 | 0.036 | 80% | M47 | 0.08 | 113% |
| M20 | 0.017 | 171% | M48 | 0.05 | 180% |
| M21 | 0.023 | 126% | M49 | 0.08 | 163% |
| M22 | 0.016 | 198% | M50 | 0.07 | 186% |
| M23 | 0.017 | 182% | M51 | 0.08 | 163% |
| M24 | 0.066 | 47% | M52 | 0.09 | 178% |
| M25 | 0.124 | 25% | M53 | 0.08 | 200% |
| M26 | 0.049 | 103% | M54 | 0.06 | 267% |
| M27 | 0.064 | 78% | M55 | 0.03 | 233% |

### Example 3 Heat Accelerated Stability Assay

### 3.1 Materials and devices

A stability test chamber (BINDER GmbH), a 0.01 mg-readability balance (METTLER TOLEDO), a pH meter (METTLER TOLEDO), a biosafety cabinet (ESCO), T2G-II small-sized motor-driven capping machine (Changsha zhongya pharmaceutical equipment co. LTD), a highspeed refrigerated centrifuge (Eppendorf), a sterilizer cabinet (Shinva Medical), Agilent 1260 high-performance liquid chromatographer, Sepax Bio-C18 4.6*250 mm 3 µm 200 Å reversed phase column, and TSKgel G2000SWXL 7.8*300 mm 3 µm were used.

### 3.2 Methods and results of the assay

The derivatives (1 mg/mL) were mixed with sodium dihydrogen phosphate (1.42 mg/mL) and dissolved in ultrapure water. After adjusting pH to about 7.4 with hydrochloric acid/sodium hydroxide, the solution was filtered into a sterilized vial in a clean bench using a 0.22 µm sterile filter. The vial was then capped and placed into the stability test chamber at 40°C. Detections were made on Days 7 (7d) and 28 (28d), and during the assay, changes in the characteristics and properties of the polypeptides were observed and recorded. The sample was then centrifuged for 3 min at 10000 rpm at 4°C, and the supernatant was transferred to a liquid phase sample vial for detecting the concentration and purity of the polypeptides using liquid phase chromatography described as below.

Conditions of the reversed phase chromatography Flow rate: 1.0 mL/min; Autosampler temperature: 15°C; Column temperature: 25°C; Detection wavelength: 214 nm; Mobile phase A: 100% H₂O + 0.05% TFA; Mobile phase B: 100% ACN; and Gradient of elution listed in Table 4:

**Table 4. Listing of the gradient of elution in the liquid phase chromatography**

| Time (min) | 0 | 3 | 8 | 13 | 14 | 20 |
|---|---|---|---|---|---|---|
| B% | 25 | 25 | 70 | 70 | 25 | 25 |

Results were shown below in Table 5.

**Table 5. Purification changes in the heat accelerated stability testing**

| | Purity from the reversed phase chromatography-214 nm | | | | |
|---|---|---|---|---|---|
| ID | 0d | 7d | 28d | Change (7d-0d) | Change (28d-0d) |
| Cagrilintide | 90.04 | 82.03 | 73.89 | -8.01 | -16.15 |
| M2 | 85.15 | 85.16 | 85.44 | 0.01 | 0.29 |
| M4 | 95 | 94.22 | 92.25 | -0.78 | -2.75 |
| M5 | 92.36 | 91.35 | 91.34 | -1.01 | -1.02 |
| M6 | 91.52 | 90.98 | 92.33 | -0.54 | 0.81 |
| M7 | 93 | 91.39 | 91.53 | -1.61 | -1.47 |
| M8 | 86.93 | 86.57 | 87.31 | -0.36 | 0.38 |
| M9 | 88.15 | 89.62 | 90.99 | 1.47 | 2.84 |
| M10 | 94.73 | 94.89 | 93.8 | 0.16 | -0.93 |

| | | | | | |
|---|---|---|---|---|---|
| Note: change (7d-0d) = purity on 7d-purity on 0d, and change (28d-0d) = purity on 28d-purity on 0d. | | | | | |

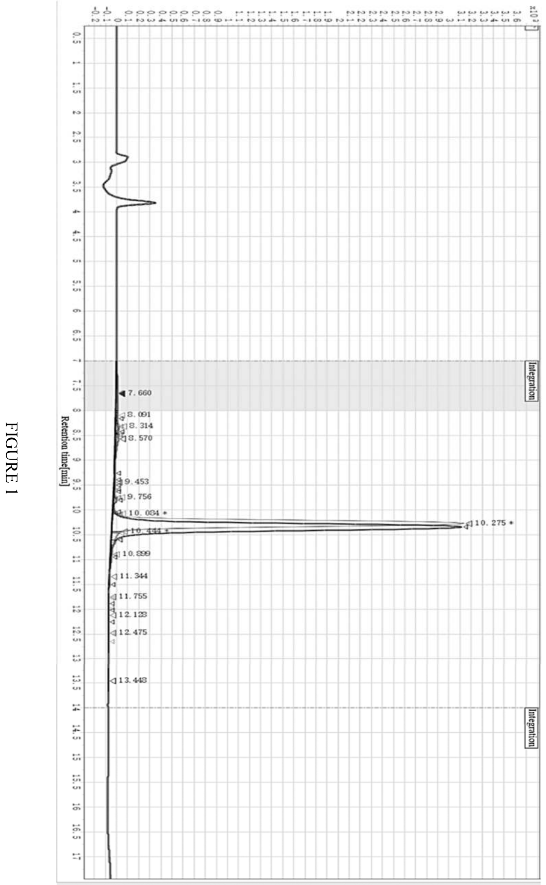

Stability data of M6 was presented specifically in FIG. 1, which indicated that after 7 days or even 1 month of heat acceleration, the spectra of reversed phase chromatography of M6 exhibited almost no change, and the spectra pre- and post-heating substantially overlapped.

The derivatives (1 mg/mL) were mixed with sodium dihydrogen phosphate (1.42 mg/mL) and dissolved in ultrapure water. After adjusting pH to about 7.4 with hydrochloric acid/sodium hydroxide, the solution was filtered into a sterilized vial in a clean bench using a 0.22 µm sterile filter. The vial was then capped and placed into the stability test chamber at 40°C. Detections were made on 10d and 20d, and during the assay changes in the characteristics and properties of the polypeptides were observed and recorded. The sample was then centrifuged for 3 min at 10000 rpm at 4°C, and the supernatant was transferred to a liquid phase sample vial for detecting the concentration and purity of the polypeptides using liquid phase chromatography described as below.

### Conditions of the reversed phase chromatography

Flow rate: 1.0 mL/min; Autosampler temperature: 25°C; Column temperature: 25°C; Detection wavelength: 214 nm; Mobile phase A: 110 mM phosphate buffer (pH 5.8):ACN = 90:10; Mobile phase B: ACN:IPA:H₂O = 85:5:10; and Gradient of elution listed in Table 6:

**Table 6. Listing of the gradient of elution in the liquid phase chromatography**

| | | | | | | |
|---|---|---|---|---|---|---|
| Time (min) | 0 | 3 | 33 | 43 | 50 | 55 |
| B% | 28 | 28 | 89 | 89 | 28 | 28 |

Results were shown below in Table 7.

**Table 7.**

| ID | Reduction in purity (%) | |
|---|---|---|
| | 10d | 20d |
| Cagrilintide | 44.83 | NA |
| M41 | 2.93 | 4.26 |
| M42 | 4.8 | NA |
| M43 | 3.6 | 6.6 |
| M44 | 21.09 | 37.35 |
| M45 | 6.54 | NA |
| M46 | 8.69 | NA |
| M47 | 9.25 | NA |
| M48 | 8.61 | NA |
| M49 | 8.5 | NA |
| M50 | 7.76 | 14.46 |
| M51 | 23.4 | 35.42 |
| M52 | 7.91 | 15.42 |
| M53 | 11.82 | 19.98 |
| M54 | 11.67 | 20.56 |

Results of the heat stability assay revealed that the polypeptide molecules of the present application exhibited remarkably superior stability as compared with the control group of the Cagrilintide molecule.

### Example 4 Animal Study

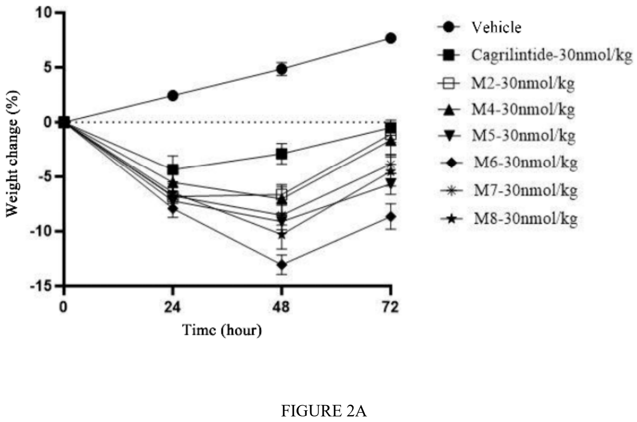

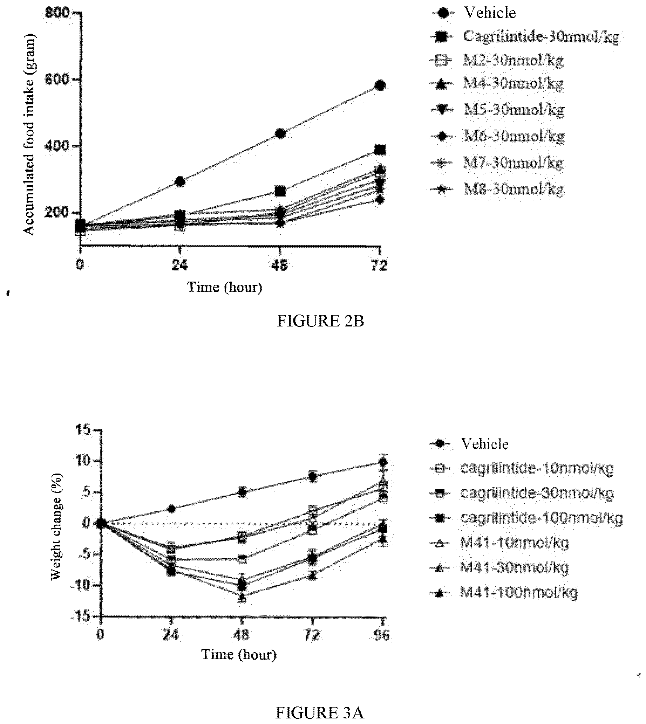

SPF male SD rats (7-8 weeks, 200-250 g) were employed in this study after health inspection and quarantine. The experimental animals were exposed to a room temperature of 20°C-23°C and a relative humidity of 40%-50%, and were provided with Co60 mouse reproduction feed 1035 and purified water supplied with water feeding bottles for free intake throughout the health inspection and quarantine and the study. Based on their mean body weight, 5 rats were assigned to each testing group for the derivatives. The rats were subcutaneously administered the derivatives at a dose of 30 nmol/kg or a vehicle (PBS pH7.4), and the dosing timepoint was recorded for each group. After dosing, the rats were put back into cages where they lived and could have access to food and water. Food consumption and weight change of the rats were recorded online or manually every 24 hours. Results for the animal study were shown in FIGs. 2A and 2B.

Specific results presented in FIGs. 2A and 2B showed that the candidate molecules of the present application have significantly higher activity than Cagrilintide, among which M6 was the most active one with an activity notably higher than Cagrilintide.

### Example 5 Efficacy Assay in SD Rats-A Dose-dependent Study

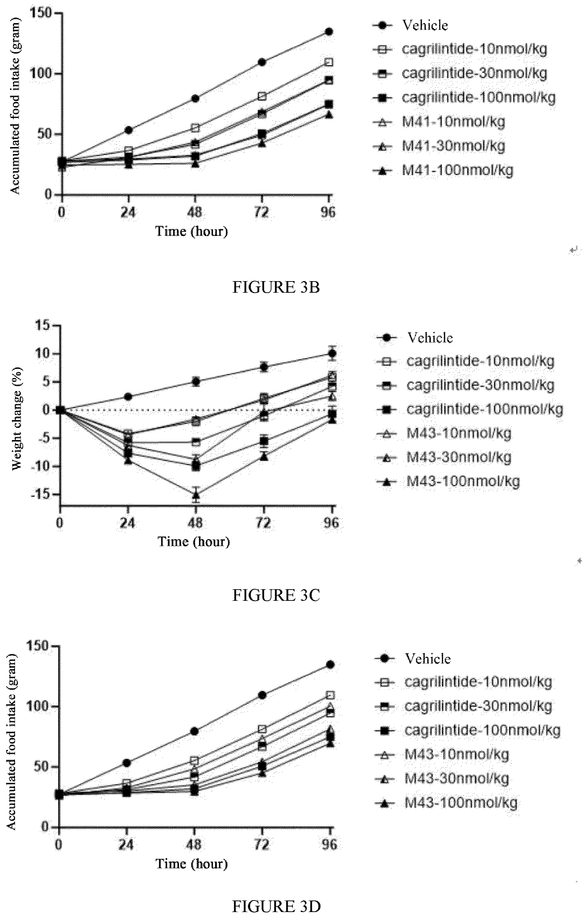

SPF male SD rats (7-8 weeks, 220-230 g) were employed in this study after health inspection and quarantine. The experimental animals were exposed to a room temperature of 20°C-23°C and a relative humidity of 40%-50%, and were provided with Co60 mouse reproduction feed 1035 and purified water supplied with water feeding bottles for free intake throughout the health inspection and quarantine and the study. Based on their mean body weight, the animals were assigned to 10 groups (designated as A1-A10) with n=5, namely the Vehicle group, the Cagrilintide groups of varying doses (high, medium, and low doses), the M41 groups of varying doses (high, medium, and low doses), and the M43 groups of varying doses (high, medium, and low doses). The compounds and dosages used in the treatment groups of the study were shown in Table 8. Using a single dose administered via subcutaneous injection, the study lasted for a total of 6 days with the day of administration designated as Day 1/Time 0 (T0), wherein on Day 0, the initial body weight of the animals were recorded and the initial feed was added, and on Day 1-Day 5, the body weight of the studied animals as well as the remaining food were recorded daily for calculating the percentage of weight change and the amount of food intake, any anomalies during which were required to be recorded and reported. During the study, the rats were in good condition and no anomaly was observed. Weight change= (BWTₙ-BWT₀)/BWT₀*100%, wherein BW represented the value of body weight, and T₀ and T₀ represented the time points of dosing and n hours post dose respectively. Dots in the figures were represented utilizing mean ± standard error of the mean (SEM). Accumulated food intake was the total amount of food intake for each of the animals prior to a certain time point post dose. The dose-effect relationship of the molecules was shown in FIGs. 3A-3D.

**Table 8. Active ingredients and dosages for testing in the dose-dependent study**

| Group | Active ingredient and dosage |
|---|---|
| A1 | Vehicle |
| A2 | Cagrilintide (10 nmol/kg) |
| A3 | Cagrilintide (30 nmol/kg) |
| A4 | Cagrilintide (100 nmol/kg) |
| A5 | M41 (10 nmol/kg) |
| A6 | M41 (30 nmol/kg) |
| A7 | M41 (100 nmol/kg) |
| A8 | M43 (10 nmol/kg) |
| A9 | M43 (30 nmol/kg) |
| A10 | M43 (100 nmol/kg) |

Results in FIGs. 3A-3D showed that both M41 and M43 dose-dependently reduced the body weight and food intake of the animals and performed better than Cagrilintide at the equivalent dosage in these regards.

### Example 6 Efficacy Assay in DIO SD Rats-A Dose-dependent Study

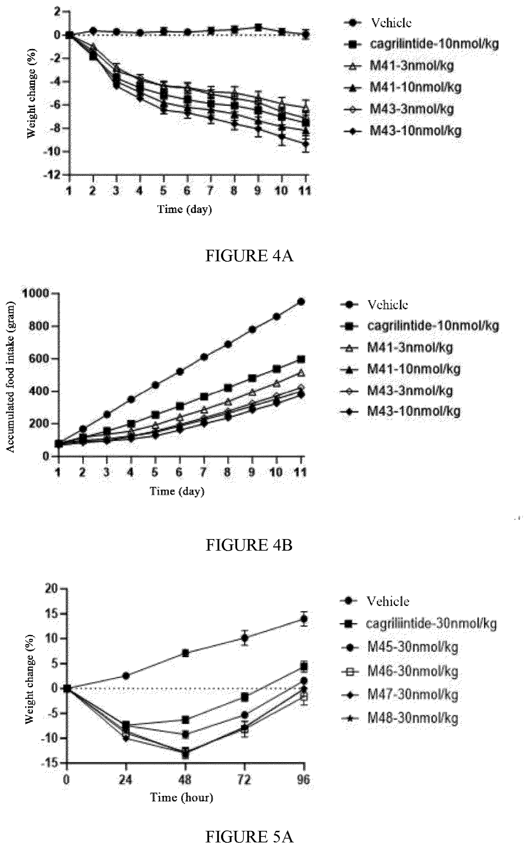

SPF male DIO SD rats (36-37 weeks, 680-110 g, fed with high-fat diet for 34 weeks from 3-week age on) were employed in this study. The experimental animals were exposed to a room temperature of 20°C-23°C and a relative humidity of 40%-50%, and were provided with high-fat diet D12492 and purified water supplied with water feeding bottles for free intake throughout the construction of the DIO animal model and the study. Based on their mean body weight, the animals were assigned to 6 groups designated as B1-B6 (n=5). The active ingredients and dosages of the compounds administered in different groups of the study were shown in Table 9. Administration via subcutaneous injection was conducted once a day for 10 continuous days, wherein the first day of administration was designated as Day 1 (D1), and on Day 0, the initial body weight of the animals were recorded and the initial feed was added, and on Day 1-Day 11, the body weight of the studied animals as well as the remaining food were recorded daily for calculating the percentage of weight change and the amount of food intake, any anomalies during which were required to be recorded and reported. During the study, the rats were in good condition and no anomaly was observed. Weight change= (BWDₙ-BWD₁)/BWD₁*100%, wherein BW represented the value of body weight, and D₁ and Dₙ represented the first and Nₜₕ days of administration respectively. Dots in the figures were represented utilizing mean ± standard error of the mean (SEM). Accumulated food intake was the total amount of food intake for animals of each group prior to a certain time point post dose. The effect on weight loss and food intake inhibition in the DIO rat model was shown in FIGs. 4A and 4B.

**Table 9. Active ingredients and dosages for testing in the efficacy assay in DIO SD rats-a dose-dependent study**

| Group | Active ingredient and dosage |
|---|---|
| B1 | Vehicle |
| B2 | Cagrilintide (10 nmol/kg) |
| B3 | M41 (3 nmol/kg) |
| B4 | M41 (10 nmol/kg) |
| B5 | M43 (3 nmol/kg) |
| B6 | M43 (10 nmol/kg) |

Results in FIGs. 4A and 4B showed that in the DIO rat model, both M41 and M43 dose-dependently reduced the body weight and food intake of the animals and performed better than Cagrilintide at the equivalent dosage in these regards.

### Example 7 Efficacy Assay in SD Rats-Mono-dose screening

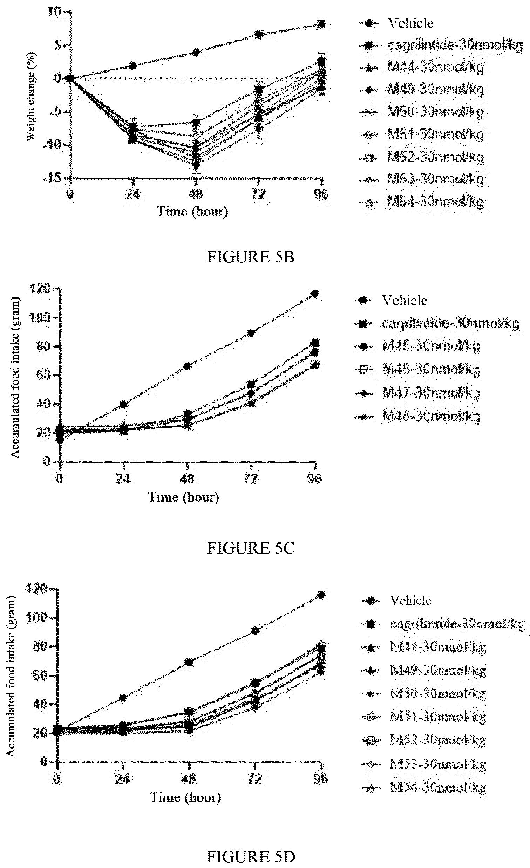

SPF male SD rats (7-8 weeks, 220-230 g) were employed in this study after health inspection and quarantine. The experimental animals were exposed to a room temperature of 20°C-23°C and a relative humidity of 40%-50%, and were provided with Co60 mouse reproduction feed 1035 and purified water supplied with water feeding bottles for free intake throughout the health inspection and quarantine and the study. The mono-dose screening assay were conducted twice sequentially, in which the animals were respectively assigned to groups of n=5 designated as C1-C6 and D1-D9 based on their mean body weight. The compounds and dosages used in the treatment groups of the study were shown in Table 10. Using a single dose administered via subcutaneous injection, the study lasted for a total of 6 days with the day of administration designated as Day 1/Time 0 (T0), wherein on Day 1-Day 5, the body weight of the studied animals as well as the remaining food were recorded daily for calculating the percentage of relative weight change and the amount of food intake, any anomalies during which were required to be recorded and reported. During the study, the rats were in good condition and no anomaly was observed. Weight change= (BWTₙ₋BWT₀)/BWT₀*100%, wherein BW represented the value of body weight, and T₀ and Tₙ represented the time points of dosing and n hours post dose respectively. Dots in the figures were represented utilizing mean ± standard error of the mean (SEM). A comparison of efficacies of different molecules was shown in FIGs. 5A-5D.

**Table 10. Active ingredients and dosages for testing in the mono-dose screening assay**

| Assay C | | Assay D | |
|---|---|---|---|
| Group | Active ingredient and dosage | Group | Active ingredient and dosage |
| C1 | Vehicle | D1 | Vehicle |
| C2 | Cagrilintide (30 nmol/kg) | D2 | Cagrilintide (30 nmol/kg) |
| C3 | M45 (30 nmol/kg) | D3 | M44 (30 nmol/kg) |
| C4 | M46 (30 nmol/kg) | D4 | M49 (30 nmol/kg) |
| C5 | M47 (30 nmol/kg) | D5 | M50 (30 nmol/kg) |
| C6 | M48 (30 nmol/kg) | D6 | M51 (30 nmol/kg) |
| | | D7 | M52 (30 nmol/kg) |
| | | D8 | M53 (30 nmol/kg) |
| | | D9 | M54 (30 nmol/kg) |

Results in FIGs. 5A-5D revealed that the efficacy of the polypeptides and derivatives thereof in the present application was comparable or superior to Cagrilintide.

### Example 8 Pharmacokinetic Study in SD Rats

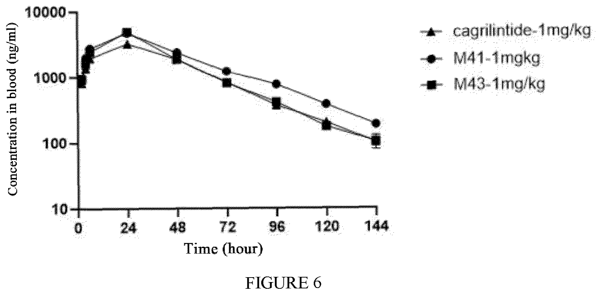

After passing the health inspection and quarantine, SPF experimental animals (male, 6-8 weeks, 200-240 g) were assigned to 3 groups (n=3), namely, the M41 group, the M43 group, and the Cagrilintide group, based on their mean body weight. The animals received a subcutaneous injection of corresponding compounds for testing (1 mg/kg and 5 ml/kg), and the day of administration was designated as D1. The whole blood of the animals was collected pre dose (-10 min) on D1 and post dose at D1-2h, D1-4h, D1-6h, D2-24h, D3-48h, D4-72h, D5-96h, and D6-120h and was processed into plasma (using EDTA for anticoagulation). Liquid chromatography-tandem mass spectrometry (LC-MS/MS) was used to detect the concentration of drug molecules in the plasma, and GraphPad Prism 10 was utilized to plot a concentration-time curve for the drugs whose pharmacokinetic parameters including Cmax, Tmax, T1/2, AUC, and MRT were then calculated with PKSolver. The concentration of different molecules in plasma over time was shown in FIG. 6. A summary of the experimental results was listed in Table 11.

**Table 11. A comparative summary of results for different molecules**

| Parameter | Unit | Cagrilintide | M41 | M43 |
|---|---|---|---|---|
| T_{1/2} | h | 24.7 | 23.7 | 21.3 |
| Tₘₐₓ | h | 24.0 | 24.0 | 24.0 |
| Cₘₐₓ | ng/mL | 3272.7 | 4784.0 | 4930.8 |
| AUC₀₋ₜ | h*ng/mL | 172880.2 | 250219.8 | 213555.4 |
| AUCp_{-inf_obs} | h*ng/mL | 176517.8 | 256644.0 | 216800.6 |
| MRT_{0-inf_obs} | h | 43.4 | 45.9 | 39.0 |

FIG. 6 and data from Table 11 demonstrated that the polypeptide molecules M41 and M43 showed an evidently higher bioavailability than Cagrilintide.

Although the present invention has been disclosed as above with the examples, those examples are not intended for limiting the invention. Appropriate changes and modifications may occur to any one of ordinary skill in the art without departing from the spirit and scope of the present invention. Thus, the scope claimed in this invention shall be defined by the claims.

## Claims

1. A derivative of a polypeptide or a pharmaceutically acceptable salt thereof, comprising an amino acid sequence of ASX₃LS TAX₈X₉X₁₀ RLADF LRHX₁₉X₂₀ X₂₁X₂₂X₂₃X₂₄X₂₅ ILPPT NVGSN TX₃₇-NH₂,
wherein:
X₃ is E or Q;
X₈ is A or V;
X₉ is L or T;
Xiₒ is G or Q;
X₁₉ is S or F;
X₂₀ is S or T;
X₂₁ is N, Q, E, S, T, A, G, H, K, R or D;
X₂₂ is N, Q, E, S, NMeAsn, αMeAsn, NMeAsp or R;
X₂₃ is L or D;
X₂₄ is K or R;
X₂₅ is P or D; and
X₃₇ is P or trans-Hyp; and
wherein the derivative comprises a fatty acid-containing side chain linked to the N terminus of the polypeptide.

2. The derivative or a pharmaceutically acceptable salt thereof of claim 1, wherein,
X₃ is Q;
preferably, X₈X₉X₁₀ is selected from the group consisting of ALG, VLG, VTQ, ATQ, and VLQ; and further preferably, X₈X₉X₁₀ is ATQ; and
more preferably,
X₁₉ is F, X₂₀ is T, X₂₃ is D, X₂₄ is R, X₂₅ is D, and X₃₇ is P; and further preferably, X₂₁ is D and/or X₂₂ is R;
or X₁₉ is S, X₂₀ is S, X₂₃ is L, X₂₄ is K, X₂₅ is P, and X₃₇ is trans-Hyp, and further preferably, X₂₁ is N, Q, E, S, T, A, G, H, K, or R and/or X₂₂ is N, Q, E, S, NMeAsn, αMeAsn, or NMeAsp.

3. The derivative or a pharmaceutically acceptable salt thereof of claim 1 or 2, wherein the sequence of the polypeptide comprises any one of sequences of SEQ ID No. 1 to SEQ ID No. 25.

4. The derivative or a pharmaceutically acceptable salt thereof of any one of claims 1-3,
the fatty acid-containing side chain being in the form of Z1+Z2+Z3,
wherein Z1 is a C16-C22 fatty diacid;
Z2 is selected from any one of γGlu, αGlu, βAsp, αAsp, Inp, and Trx, or is absent;
Z3 is 0-6 AEEA(s), for example 0, 1, 2, 3, 4, 5, or 6, AEEA(s); and
wherein Z1, Z2, and Z3 are linked via amide bonds.

5. The derivative or a pharmaceutically acceptable salt thereof of claim 4, wherein the derivative of the polypeptide is selected from M1 to M55.

6. A polypeptide comprising the amino acid sequence involved in any one of claims 1-5.

7. A pharmaceutical composition comprising the derivative and a pharmaceutically acceptable salt thereof of any one of claims 1-5 or the polypeptide of claim 6, and a pharmaceutically acceptable excipient.

8. Use of the derivative and a pharmaceutically acceptable salt thereof of any one of claims 1-5 or the polypeptide of claim 6 in the manufacture of a medicament for preventing and/or treating overweight and/or obesity and/or type I or type II diabetes and/or osteoporosis and/or neuropathic pain.

9. Use of the derivative and a pharmaceutically acceptable salt thereof of any one of claims 1-5 or the polypeptide of claim 6 in reducing food intake.

10. A method of preventing and/or treating overweight and/or obesity and/or type I or type II diabetes and/or osteoporosis and/or neuropathic pain, comprising administering to a subject a prophylactically or therapeutically effective amount of the derivative and a pharmaceutically acceptable salt thereof of any one of claims 1-5, the polypeptide of claim 6, or the pharmaceutical composition of claim 7.

11. A method of reducing food intake, comprising administering to a subject a prophylactically or therapeutically effective amount of the derivative and a pharmaceutically acceptable salt thereof of any one of claims 1-5, the polypeptide of claim 6, or the pharmaceutical composition of claim 7.

12. A method of preventing and/or treating an amylin receptor related disease or a lipid metabolism disorder, comprising administering to a subject a prophylactically or therapeutically effective amount of the derivative and a pharmaceutically acceptable salt thereof of any one of claims 1-5, the polypeptide of claim 6, or the pharmaceutical composition of claim 7.
